# EUROPEAN PATENT SPECIFICATION

(11) **EP 0 711 155 B1**
(45) Date of publication and mention of the grant of the patent: **22.03.2000**
(21) Application number: 94920632.0
(22) Date of filing: 01.07.1994
(51) Int. Cl.: A61K 31/195, A61K 31/20

(54) **AMINO ACID SUPPLEMENT**
AMINOSÄUREERGÄNZUNG
COMPLEMENT D'ACIDES AMINES

(30) Priority: 02.07.1993 SE 9302296
(43) Date of publication of application: 15.05.1996
(73) Proprietor: Fresenius Kabi AB, 751 74 Uppsala (SE)
(72) Inventor: LINDGREN, Svante, S-195 00 Märsta (SE); ALHADEFF, Paul, S-112 65 Stockholm (SE); JONSSON, Uno, S-194 36 Upplands Väsby (SE); ROSENKVIST, Kenneth, S-184 61 Akersberga (SE); SANDBERG, Göran, S-762 00 Rimbo (SE); SKOGHOLM, Clas, S-129 40 Hägersten (SE); WAHLEN, Raymond, S-194 53 Upplands Väsby (SE)
(74) Representative: Luderschmidt, Schüler & Partner GbR
(86) International application number: SE9400655
(87) International publication number: WO9501172

(56) References cited:
- WO-A-91/09837
- STN INTERNATIONAL, File Embase, Embase accession no. 94020359, LOZANO P. et al.: "Synthesis of L-tyrosine glyceryl ester catalyzed by .alpha.-chymotrypsin in water-miscible organic solvents: A possible sun-tan accelerator products"; & BIOTECHNOL. LETT., (1993), 15/12, (1223-1228).
- PATENT ABSTRACTS OF JAPAN, Vol. 13, No. 24, C-56; & JP,A,63 230 630 (NIPPON OIL & FATSCO LTD), 27 Sept 1988.
- PATENT ABSTRACTS OF JAPAN, Vol. 14, No. 220, C-717; & JP,A,2 053 724 (NIPPON OIL & FATS CO LTD), 22 February 1990.
- STN INTERNATIONAL, File Medline, Medline accession no. 90061402, WRETLIND A: "Future perspectives in parenteral nutritiona. Zukunftsperspektiven in der parenteralen Ernahrung"; & INFUSIONSTHERAPIE, (Oct 1989), 16 (5), 192-7, Ref: 4.

## Description

### Field of invention

The present invention relates to preparations to be used in therapy or nutrition containing stabilized or solubilized forms of amino adds otherwise unstable or poorly soluble in aqueous fluids. The said amino adds are present as glyceryl esters of compounds chosen among at least one amino acid and fatty adds.

### Background of the invention

The present invention intends to increase the possibility to add chemically and physically sensitive or poorly soluble amino adds to a solution capable of being stored without decomposition.

An important aspect of the invention is to provide preparations of amino adds useful in therapy and nutrition which can be readily processed to conventional "all-in-one" nutritional compositions containing e.g. lipids with essential fatty adds, free amino adds, carbohydrates, electrolytes, vitamins, trace elements and technical auxiliary substances.

Examples of a poorly soluble and/or unstable, but nutritionally and therapeutically important amino adds, are tyrosine, cystein, glutamine and the branched-chain amino adds. The poor solubility of many amino adds limits their usefulness in amino add solutions and new concepts have to be found in order to increase their applicability.

Another problem related to amino adds is that many of them are decomposed in water solutions, mixtures or emulsions, especially during pasteurization or hot sterilization. Most amino adds are decomposed to a small degree in aqueous solutions, but certain amino acids as for example glutamine, asparagine, cysteine and lysine are decomposed to a larger degree (K.Khan et al in "The stability of glutamine in total parenteral nutrition solutions". Clin. Nutrition, Vol. 10, pages 193-198, 1991 and K.Khan and M. Elia in "Factors affecting the stability of L-glutamine in solution". Clin. Nutrition, Vol. 10, pages 186- 192, 1991).

Lysine, in the combination with heat and reducing sugars (as glucose), is one of the combinations in the so called Maillard reaction which produces nutritionally unavailable products, see J. Adrian, Nutritional and Physiological Consequences of the Maillard Reaction, World Review of Nutrition and Dietics, Vol.19, pp 71-122 (Karger, Basel 1974)

Many attempts have been made to comply with these mentioned problems. For example peptides have been used instead of the amino adds. Oligopeptides mainly dipeptides have been used to stabilize and increase the solubility of branched-chain amino adds, glutamine and tyrosine but solubility problems of the peptides might arise and furthermore the peptides are complicated and expensive to synthesise. This technique is disdosed in WO 92/09277 which describes a balanced composition of glutamine containing free glutamine in combination with a peptide derivative thereof and alpha-ketoglutarate as a glutamine precursor. An alternative method is to dissolve the glutamine as an N-acetyl compound. Another possibility is to lyophilize glutamine and prior to use dissolve it in sterile water or an amino add solution or a glucose solution.

The supplement of glutamine in the form of an N-acetyl derivative provides a stable product, but it has a very low biological availability with a urinary excretion of more than 50% of the unmetabolized products for a given dose. Furthermore, these products are very acidic and demand supplies of alkalis to adjust the pH-value of the composition. A possible explanation to the increased urinary excretion is that N-acetylated amino adds are deacetylated in the kidneys in man, which allows the substrates to be accumulated and consequently an increased amount will be excreted via the urine.

It is previously disdosed in the International patent application WO 91/09837 to link peptides or amino adds to fats by means of a spacing group. These conjugates are more laborious to assemble than the compounds according to the present invention and they will require a more complicated hydrolysis procedure *in-vivo* to be decomposed to amino adds and free fatty adds or glycerides. Furthermore, by using a spacing group in a parenteral composition the patient will be supplied with surplus exogenous compounds, which is generally avoided in this technology. Amino adds directly coupled to fatty adds are earlier known to be surface active (see e.g. Surface active agents, their chemistry and technology by A.M. Schwartz and J.W. Perry, Interscience Publishers Inc. New York 1949), but the utilization of such compounds in nutritionally completed and balanced products as emulsifying agents, as well as a source of sensitive or poorly soluble amino adds, as for example glutamine, asparagine, tyrosine, cystein and lysine, is a novel concept.

In accordance with the present invention it is found that by coupling of an amino add to glycerol or to a glyceride, compounds are formed which are chemically and physically more resistant in the environment present in the manufacture of products for enteral and parenteral use. The resulting compounds will also enable an increased addition of poorly soluble amino adds, such as glutamine and tyrosine, to nutrient fluids. Such an increased supply of for example glutamine is required to reach therapeutically effective levels in certain important applications.

### Description of the invention

The present invention is related to preparations useful in oral, enteral or parenteral therapy and/or nutrition, when delivering sensitive and/or poorly soluble amino acids containing glyceryl esters of amino adds. The said amino adds will be bound by an ester linkage to a mono- or a diglyceride of a fatty acid or to glycerol. The fatty adds will have between 6 and 24 carbon atoms. The amino adds are preferably essential and most preferably, either poorly soluble or unstable during the preparation or unstable during storage in liquid preparations intended for parenteral or enteral administration. Especially preferred amino adds are selected from a group consisting of glutamine, lysine, tyrosine, arginine, asparagine and cysteine, but it is within the scope of the invention to include any amino acid in the preparation and especially essential amino adds. The preparations can have surface active properties and may be included in surfactant compositions.

The present invention is also related to lipid emulsions and compositions containing the said preparations and conventional additives and supplements used in the field of enteral or parenteral nutrition.

An important object of the invention is to provide a method to improve the stability and/or the solubility of amino adds otherwise difficult to handle in solutions, by linking to glycerol or to mono-, di- or triglycerides of fatty acids having between 6 and 24 carbon atoms.

Another object of the invention is the use of the mentioned preparation for the manufacture of a liquid product suitable for heat treatment and storage.

The preparations according to the present invention can contain any combination of:
a) glycerol with one, two or three amino acids bound with ester linkages in each molecule;
b) monoglycerides of fatty acids with one or two amino acids bound with ester linkages in each molecule or
c) diglycerides of fatty acids with one amino acid bound with an ester linkage in each molecule.
Accordingly, the inventive preparations can include compounds that either are mono-, di- or triglycerides with respect to the amino acids and mono- or diglycerides concerning the fatty adds. A monoglyceride with only one amino acid bound to the glycerol is therefore possible, for example monoglutamine-glycerol. Any of these categories of compounds is an alternative within the present invention and the choice will be directed by several considerations regarding the preparation qualities and the final product which will contain such a preparation. Such important considerations are the emulsifying capacity of the preparation, the osmolality increasing capacity and the use of the final product as a total parenteral nutrition (TPN)-composition. It may also be possible to direct the distribution and to control the release of the amino acids in-vivo, by a careful selection of the glycerol esters of amino acids, in relation to the substituents of these compounds, which are delivered by the preparation.

These glyceryl esters of amino acids are suitable as a source of amino acids with nutritional and/or therapeutical value beside their capacity of stabilizing the amino adds.
The glyceryl esters of amino acids have been shown to be stable in a water solution subjected to autoclavation or pasteurization and they can act as emulsifiers in conventional emulsion systems consisting of oil in water, alone or together with commercially used and clinically acceptable emulsifiers, such as the egg yolk phospholipids found in conventional nutritional emulsions, e.g. Intralipid®.

The choke of amino adds to be linked to the glycerides or to glycerol is guided by the need to have an unstable or poorly soluble amino acid incorporated and stabilized or solubilized. Examples of suitable amino acids are cysteine, glutamine, tyrosine and asparagine.

The synthesis of the glycerides of at least one amino acid can be performed in three alternative ways:
1) All-synthetic synthesis with raw materials made from synthetic mono-and diglycerides, which are coupled to amino acids by means of DCC (di-cyclo-hexylcarbodiimid) and DMAP (di-methyl-amino-pyridine).
2) Biochemically synthezised glycerides where the amino adds and/or the fatty acids are interesterified in 1-and/or 2- and/or 3-position on the glycerol backbone with the help of specific lipases. We have shown that amino add ethyl esters are interesterified with for example triolein by means of a commercial lipase (Lipozyme® Novo Industries Copenhagen Denmark) having the specificity for 1-and 3-positions in the glyceride. The enzymes are immobilized and can therefore be reused several times. Other lipases with other specificities are possible to use for the synthesis.
3) Semisynthetically, where the starting material is a mono- or diglyceride or mixtures of them, synthetized enzymatically by means of lipase and an oil or fat of natural origin, for example soy bean oil or another vegetable oil or fat of animal or marine origin.

The fatty adds to be incorporated and delivered with the above mentioned preparations preferably have a chain length long enough to provide surface active properties to the preparations. The most preferred fatty acids are the long chain fatty acids belong to the w3-, w6- and w9-series.

The inventive preparations containing glyceryl esters of amino acids are readily miscible with other conventional components used in parenteral or enteral nutrition such as lipids, solutions of free amino acids (if necessary or desired), carbohydrates and other technically auxiliary constituents e.g. emulsifiers and isotonicity adjusters.
Besides the choice of amino acids in the preparations the fatty adds can be chosen to have an appropriate balance between essential and non-essential constituents or when desired chosen to be a supplement of a certain beneficial fatty acid. The said preparations can due to their surface active properties be used as emulsifiers, if necessary with the addition of conventional phospholipids, when preparing lipid emulsions.

It is especially preferred to indude the preparations containing the aforedescribed glyceryl esters of amino adds in compositions, wherein all the main nutrient ingredients are present, suitable for oral, enteral or parenteral feeding, with a possibility to admix electrolytes, trace elements and vitamins. Lipid emulsions containing the inventive preparation are surprisingly suitable for further addition of vitamins, electrolytes and trace elements to obtain total parenteral nutrition (TPN) compositions because of its increased physical and chemical stability. In conventional TPN-mixtures the normally dissociated and heavily charged amino acids will influence the stability of an emulsion. The stability will often be very limited when other compounds contributing to electrolyte level of the aqueous phase are added to such an emulsion.

When using preparations according to the present invention, wherein the amino acids (or a substantial part thereof) are incorporated in amphiphilic molecules, the bound amino acids will be enriched in the interface between the lipid droplets and the aqueous phase of the emulsion. This condition leads to a reduction of the level of dissociated amino acids in the aqueous phase and thereby an increased physical stability of emulsions containing amino acids according to the present invention. To obtain such an increased stability in a TPN-mixture is a significant improvement, when considering that electrolytes, trace elements and vitamins can be added at an earlier stage before administration. A stability for such a TPN-mixture exceeding 96 hours will therefore be obtained by means of a preparation according to the invention.

The preparations and compositions according to the present invention will be advantageous for supplying otherwise poorly soluble and unstable amino acids in nutritionally and/or therapeutically suitable amounts in oral, enteral or parenteral compositions. The preparations will also contribute to an enhanced stability of emulsions and especially such emulsions which are intended for total parenteral nutrition. Another important advantage of the present invention is that Maillard reactions during heat treatment of TPN preparations can be avoided, in those cases such reactions conventionally might be present. This will largely facilitate the choice of containers for storing the compositions according to the invention, by making it possible to sterilize such a product in its final container.

The following examples are not intended to be limiting for the scope of invention, but shall be regarded as illustrations to some functioning embodiments of the present invention.

### EXAMPLES

### Example 1.

### Preparation of a tyrosine containing triglyceride

20 grains of tyrosine ethylester were dissolved in 100 grams of triolein. 12 grams of the immobilized lipase (Lipase® Novo, Copenhagen, Denmark) was added under a stream of nitrogen, stirring and heated to 50 °C. 1,2ml distilled water was added and the mixture was reacted during two days. Thereafter the liquid was filtered off, extracted ten times with 200 ml distilled water each time and finally dried with dried sodium sulphate giving approx. 25g of oil. No more purification was done in this experiment. Quantitative analysis regarding bound tyrosine was carried out according to the following:
Tyrosine containing triglyceride (530 mg) and HCl (6 M, 10 ml) was added to a tube. The tube was evaporated by suction, stoppered and placed in an oven (110°C) over night. The water phase was evaporated and the residue was dissolved in HCl (0,1 M, 1 ml). The sample was ultrafiltrated and analysed by HPLC.
The level of tyrosine was 4 mg/ml which corresponds to 0,75 % (w/w) tyrosine in the oil.

### Example 2.

### Preparation of an arginine containing triglyceride

100 grams of triolein and 20 grains of arginylethylester were mixed and dissolved with 12 grams of immobilized lipase (Lipase® Novo, Copenhagen, Denmark) was added under a stream of nitrogen, stirring and heated to 50 °C. Distilled water, 1,2 ml, was added and the mixture was reacted during two days. Thereafter the liquid was filtered off, extracted ten times with 200 ml distilled water each time and finally dried with dried sodium sulphate giving approx. 20 g oil. No more purification of the oil was done in this experiment. Quantitative analysis regarding combined arginine was carried out according to the same manner as described above corresponding to 0,34 % (w/w) combined arginine in the oil.

## Claims

1. A preparation for delivering sensitive or poorly soluble amino adds orally enterally or parenterally in therapy and/or nutrition **characterized in that** it contains glyceryl esters of amino acids.

2. A preparation according to claim 1 **characterised in that** it contains at least one amino acid bound with an ester linkage to a mono- or a diglyceride of a fatty acid or to glycerol.

3. A preparation according to claim 1 or 2 wherein the fatty adds have between 6 and 24 carbon atoms.

4. A preparation according to claims 1 to 3 **characterized in that** the amino acids are essential amino acids.

5. A preparation according to any of claims 1 to 4 **characterized in that** the amino acids are selected from a group consisting of glutamine, lysine, tyrosine, arginine, asparagine, cysteine and branched-chain amino acids.

6. A preparation according to claims 3 to 5 **characterized in that** the fatty adds are belonging to the w3-, w6- or w9-series.

7. A surface active compostion containing the preparation according to claims 1 to 6.

8. A lipid emulsion for oral, enteral or parenteral use containing the preparation according to any of claims 1-6 or the composition according to claim 7, lipids, an aqueous phase and if necessary an additional emulsifier.

9. A composition containing the preparation according to claims 1-6 and at least one constituent selected from the group consisting of lipids, free amino adds, carbohydrates, vitamins, emulsifiers, isotonicity adjusters, electrolytes and trace elements.

10. A method of improving the stability and/or the solubility of an amino acid by linking it to glycerol or to mono-, di- or triglycerides of fatty adds having 6 to 24 carbon atoms.

11. Use of a preparation according to claims 1-7 for the manufacture of a stable liquid product, comprising the compositions of claim 9, having improved stability during heat treatment and subsequent storage.

## Patentansprüche

1. Präparat zur oralen, enteralen oder parenteralen Verabreichung empfindlicher oder schwer löslicher Aminosäuren in Therapie und/oder Ernährung, **dadurch gekennzeichnet**, dass es Glycerylester von Aminosäuren enthält.

2. Präparat gemäß Anspruch 1, **dadurch gekennzeichnet**, dass es mindestens eine mit einer Esterbindung an ein Mono- oder Diglycerid einer Fettsäure oder an Glycerin gebundene Aminosäure enthält.

3. Präparat gemäß Anspruch 1 oder 2, bei dem die Fettsäuren zwischen 6 und 24 Kohlenstoffatome aufweisen.

4. Präparat gemäß Ansprüchen 1 bis 3, **dadurch gekennzeichnet**, dass die Aminosäuren essentielle Aminosäuren sind.

5. Präparat gemäß einem der Ansprüche 1 bis 4, **dadurch gekennzeichnet**, dass die Aminosäuren aus einer Gruppe ausgewählt sind die, aus Glutamin, Lysin, Tyrosin, Arginin, Asparagin, Cystein und verzweigtkettigen Aminosäuren besteht.

6. Präparat gemäß Ansprüchen 3 bis 6, **dadurch gekennzeichnet**, dass die Fettsäuren den w3-, w6- oder w9-Reihen angehören.

7. Oberflächenaktive Zusammensetzung mit einem Gehalt an dem Präparat gemäß Ansprüchen 1 bis 6.

8. Lipidemulsion zur oralen, enteralen oder parenteralen Verwendung, enthaltend das Präparat gemäß einem der Ansprüche 1 bis 6 oder die Zusammensetzung gemäß Anspruch 7, Lipide, eine wässerige Phase und erforderlichenfalls einen zusätzlichen Emulgator.

9. Zusammensetzung, enthaltend das Präparat gemäß Ansprüchen 1 bis 6 und mindestens einen Bestandteil, ausgewählt aus der Gruppe, die aus Lipiden, freien Aminosäuren, Kohlehydraten, wie Vitamen, Emutgatoren, Mitteln zur Einstellung der Isotonizität, Elektrolyten und Spurenelementen besteht.

10. Verfahren zur Verbesserung der Stabilität und/oder der Löslichkeit einer Aminosäure durch ihre Bindung an Glycerin oder an Mono-, Di- oder Triglyceride von Fettsäuren mit 6 bis 24 Kohlenstoffatomen.

11. Verwendung des Präparats gemäß Ansprüchen 1 bis 7 zur Herstellung eines stabilen flüssigen Produkts, das die Zusammensetzungen des Anspruchs 9 umfasst und eine verbesserte Stabilität während der Wärmebehandlung und anschließenden Lagerung aufweist.

## Revendications

1. Préparation convenant pour délivrer des acides aminés sensibles ou peu solubles par voie orale, intestinale ou parentérale en thérapie et/ou en nutrition, caractérisée en ce qu'elle contient des esters de glycéryle d'acides aminés.

2. Préparation selon la revendication 1, caractérisée en ce qu'elle contient au moins un acide amine lié par une liaison ester à un mono- ou diglycéride d'un acide gras ou au glycérol.

3. Préparation selon la revendication 1 ou 2, dans laquelle les acides gras ont entre 6 et 24 atomes de carbone.

4. Préparation selon les revendications 1 à 3, caractérisée en ce que les acides aminés sont des acides aminés essentiels.

5. Préparation selon l'une quelconque des revendications 1 à 4, caractérisée en ce que les acides aminés sont choisis dans le groupe formé par la glutamine, la lysine, la tyrosine, l'arginine, l'asparagine, la cystéine et les acides aminés à chaîne ramifiée.

6. Préparation selon les revendications 3 à 5, caractérisée en ce que les acides gras appartiennent aux séries w3, w6 ou w9.

7. Composition tensio-active contenant la préparation selon les revendications 1 à 6.

8. Émulsion lipidique à usage oral, intestinal ou parentéral contenant la préparation selon l'une quelconque des revendications 1 à 6 ou la composition selon la revendication 7, des lipides, une phase aqueuse et, si nécessaire, un émulsifiant supplémentaire.

9. Composition contenant la préparation selon les revendications 1 à 6 et au moins un constituant choisi dans le groupe formé par les lipides, les acides aminés libres, les glucides, les vitamines, les émulsifiants, les agents d'ajustement d'isotonie, les électrolytes et les oligoéléments.

10. Procédé pour améliorer la stabilité et/ou la solubilité d'un acide aminé par liaison de celui-ci au glycérol ou à des mono-, di- ou triglycérides d'acides gras ayant 6 à 24 atomes de carbone.

11. Utilisation d'une préparation selon les revendications 1 à 7, pour la fabrication d'un produit liquide stable, comprenant la composition de la revendication 9, ayant une stabilité améliorée pendant un traitement thermique et une conservation subséquente.
